(19) Europäisches Patentamt
European Patent Office
Office européen des brevets

(11) Veröffentlichungsnummer: **0 226 917**
**B1**

(12) # EUROPÄISCHE PATENTSCHRIFT

(45) Veröffentlichungstag der Patentschrift: **08.03.89**

(51) Int. Cl.⁴: **C07C 69/734**, C07C 147/06, A01N 37/10, C07C 149/40

(21) Anmeldenummer: **86116987.8**

(22) Anmeldetag: **06.12.86**

(54) **Acrylsäureester und Fungizide, die diese Verbindungen enthalten.**

(30) Priorität: **20.12.85 DE 3545319**

(43) Veröffentlichungstag der Anmeldung:
**01.07.87 Patentblatt 87/27**

(45) Bekanntmachung des Hinweises auf die Patenterteilung:
**08.03.89 Patentblatt 89/10**

(84) Benannte Vertragsstaaten:
**AT BE CH DE ES FR GB GR IT LI NL SE**

(56) Entgegenhaltungen:
**EP-A- 0 015 502**
**EP-A- 0 178 826**

(73) Patentinhaber: **BASF Aktiengesellschaft,
Carl-Bosch-Strasse 38, D-6700 Ludwigshafen(DE)**

(72) Erfinder: **Schirmer, Ulrich, Dr., Berghalde 79, D-6900 Heidelberg(DE)**
Erfinder: **Karbach, Stefan, Dr., Sperlinggasse 3, D-6700 Ludwigshafen(DE)**
Erfinder: **Pommer, Ernst-Heinrich, Dr., Berliner Platz 7, D-6703 Limburgerhof(DE)**
Erfinder: **Ammermann, Eberhard, Dr., Sachsenstrasse 3, D-6700 Ludwigshafen(DE)**
Erfinder: **Steglich, Wolfgang, Prof. Dr., Hobsweg 77, D-5300 Bonn-Roettgen(DE)**
Erfinder: **Schwalge, Barbara Agnes Maria, Tannenweg 9, D-5204 Lohmar 1(DE)**
Erfinder: **Anke, Timm, Prof. Dr., Theodor-Heuss-Strasse 17, D-6750 Kaiserslautern(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die vorliegende Erfindung betrifft neue Acrylsäureester und Fungizide, welche diese Verbindungen enthalten.

Es ist bekannt, N-Tridecyl-2,6-dimethylmorpholin oder seine Salze, z.B. das Acetat, als Fungizide zu verwenden (DE 1 164 152, 1 173 722). Ihre fungizide Wirkung ist jedoch in manchen Fällen ungenügend.

Es wurde nun gefunden, daß neue Acrylsäureester der Formel

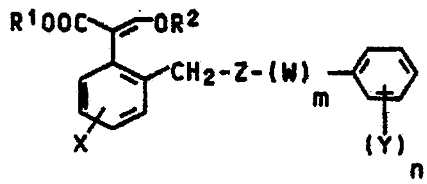

in der $R^1$ und $R^2$ unabhängig voneinander $C_1$–$C_8$-Alkyl bedeuten,
X Wasserstoff, $C_1$–$C_4$-Alkyl, Halogen, $C_1$–$C_4$-Alkoxy, Halogenalkyl, Cyano oder Nitro bedeutet,
W einen gegebenenfalls durch Alkyl substituierten, gegebenenfalls ungesättigten $C_1$–$C_{10}$-Alkylenrest bedeutet,
Y Wasserstoff, Alkyl, Halogenalkyl, Alkoxyalkyl, Cycloalkyl, Aralkyl, Aryl, Aryloxy, Halogen, eine gegebenenfalls substituierte an den Benzolrest annellierte $C_4H_4$-Kette, Alkoxy, Halogenalkoxy, Alkylthio, Thiocyanato, Cyano, $NO_2$,

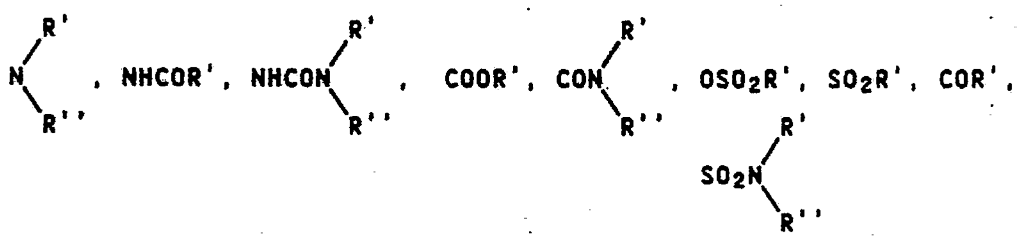

bedeutet, wobei R′ und R″ jeweils unabhängig voneinander Wasserstoff, Alkyl, Alkoxy, Alkylthio, Cycloalkyl oder gegebenenfalls durch Alkyl, Halogen oder Alkoxy substituiertes Phenyl bedeuten, m die Zahlen 0 oder 1 und n die Zahlen 1 bis 4 sowie Z Sauerstoff, Schwefel, SO oder $SO_2$ bedeutet, außer der Verbindung α-(2-Phenoxymethyl-phenyl)-β-methoxy-acrylsäuremethylester,
eine ausgezeichnete fungizide Wirkung haben.

Die in der allgemeinen Formel angeführten Reste können beispielsweise folgende Bedeutung haben:
$R^1$ bzw. $R^2$ gegebenenfalls verzweigtes $C_1$–$C_8$-Alkyl (z.B. Methyl, Ethyl, Isopropyl, n-Propyl, n-Butyl, sec.-Butyl, tert.-Butyl, iso-Butyl, sec.-Pentyl, n-Hexyl, α-Ethyl-n-hexyl, n-Octyl),
X Wasserstoff, $C_1$–$C_4$-Alkyl (z.B. Methyl, t-Butyl), Halogen (z.B. Fluor, Chlor, Brom), $C_1$–$C_4$-Alkoxy (z.B. Methoxy, n-Butoxy) oder Halogenalkyl (z.B. Trifluormethyl), Cyan, Nitro,
W einen gegebenenfalls durch $C_1$–$C_4$-Alkyl substituierten, gegebenenfalls ungesättigten $C_1$–$C_{10}$-Alkylenrest (beispielsweise Methylen, Methylmethylen, Dimethylmethylen, Propylen, Allylen, Hexylen, Ethylen, Methylethylen, Methylpropylen, Ethylpropylen, Butylen, Pentylen, Methylpentylen, Dimethylpropylen, Heptylen, Ethylbutylen, Trimethylpentylen),
Y Wasserstoff, $C_1$–$C_{12}$-Alkyl (z.B. Methyl, Ethyl, t-Butyl, Dodecyl), Halogenalkyl (z.B. Trifluormethyl), $C_1$–$C_4$-Alkoxy-$C_1$–$C_4$-alkyl (z.B. Methoxymethyl), $C_5$–$C_8$-Cycloalkyl (z.B. Cyclohexyl), Aralkyl (z.B. Benzyl), Aryl (z.B. Phenyl), Aryloxy (z.B. Phenoxy), Halogen (z.B. Fluor, Chlor, Brom oder Jod), eine gegebenenfalls substituierte $C_4H_4$-Kette, die mit dem Benzolring zu einem gegebenenfalls substituierten Naphthylring kondensiert ist, $C_1$–$C_8$-Alkoxy (z.B. Isopropoxy, Hexyloxy), Halogen-$C_1$–$C_4$-alkoxy (z.B. 1,1,2,2-Tetrafluorethoxy), $C_1$–$C_4$-Alkylthio (z.B. Methylthio), Thiocyanato, Cyano, $NO_2$,

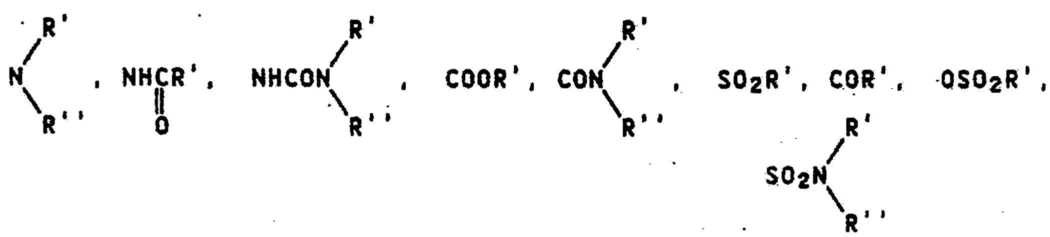

wobei R′ und R″ jeweils unabhängig voneinander Wasserstoff, $C_1$–$C_4$-Alkyl (z.B. Methyl, Ethyl), $C_1$–$C_4$-Alkoxy (z.B. Methoxy, tert.-Butoxy), $C_1$–$C_4$-Alkylthio (z.B. Methylthio), $C_5$–$C_8$-Cycloalkyl (z.B. Cyclohexyl) oder gegebenenfalls durch $C_1$–$C_4$-Alkyl, Halogen oder $C_1$–$C_4$-Alkoxy substituiertes Phenyl (z.B. Phenyl, 3-Chlorphenyl, 4-Methylphenyl, 3-Methoxyphenyl) bedeuten.

Die neuen Verbindungen können beispielsweise nach folgendem Verfahren hergestellt werden: 2-Methylphenylessigsäureester der allgemeinen Formel

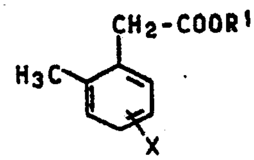

werden nach Wislicenus (Liebigs Annalen 424, 215 (1921) und Liebigs Annalen 413, 206 (1917)) mit Ameisensäuremethylester und Natriumhydrid in einem inerten Lösungsmittel zur Reaktion gebracht. Die so erhaltenen Verbindungen der allgemeinen Formel

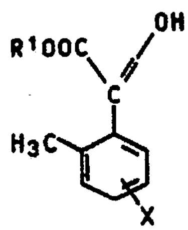

werden mit einem Alkylierungsmittel in Gegenwart einer Base in einem Lösungsmittel (z.B. Aceton) zu α-(2-Methylphenyl)-β-alkoxyacrylsäureestern umgesetzt,

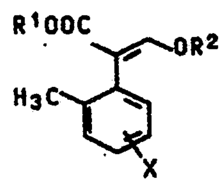

in denen $R^1$, $R^2$ und X die oben genannten Bedeutungen haben.

Die Bromierung dieser Verbindung mit N-Bromsuccinimid (Horner, Winkelmann, Angew. Chemie 71, 349 (1959) führt zu α-(2-Brommethylphenyl)-β-alkoxy-acrylsäureestern der allgemeinen Formel

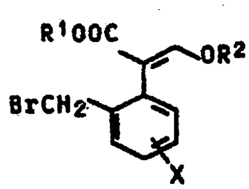

in der $R^1$, $R^2$ und X die oben genannten Bedeutungen haben.

Die oben genannten Brommethylverbindungen werden mit Alkoholen, Phenolen bzw. Mercaptanen zu den neuen Verbindungen (Z = Sauerstoff oder Schwefel) umgesetzt:

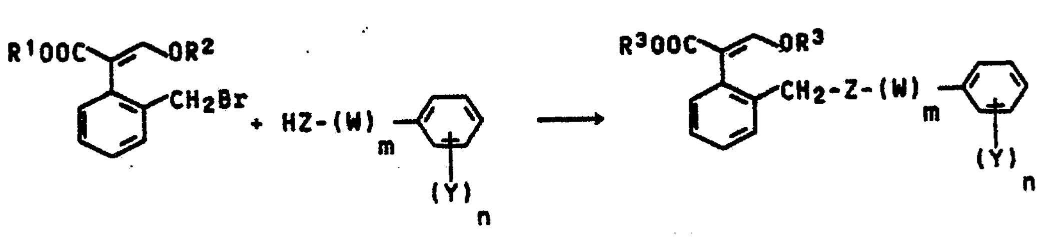

in denen $R^1$, $R^2$, X, W, Y, m und n die oben genannten Bedeutungen haben (vgl. Houben-Weyl, Methoden der organischen Chemie VI/3, 54 ff (1965)).

Die neuen Verbindungen mit Z gleich –SO– oder –$SO_2$– erhält man durch Oxidation der entsprechenden –S-Verbindungen:

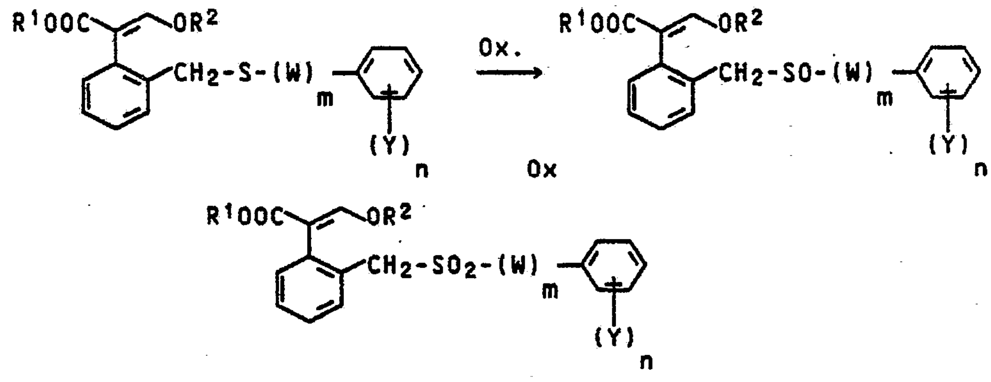


(vgl. Houben-Weyl, Methoden der Organischen Chemie IX, 213, 228 (1955)).
Die nachstehenden Vorschriften erläutern die Synthese der Ausgangsprodukte.

Vorschrift A

α-(2-Methylphenyl)-β-methoxy-acrylsäuremethylester

16,5 g 2-Methylphenylessigsäuremethylester werden in 10 ml Ameisensäuremethylester gelöst und langsam zu einer Suspension aus 3 g Natriumhydrid in 150 ml abs. Ether getropft. Nach 4 h Rückfluß wird mit verdünnter HCl angesäuert, die org. Phase abgetrennt, mit Wasser gewaschen, über $MgSO_4$ getrocknet und eingeengt. Man erhält 13,8 g eines hellgelben Öles (α-Formyl-(2-methylphenyl)essigsäuremethylester), das zusammen mit 5,8 ml Dimethylsulfat, 10,9 g Kaliumcarbonat und 70 ml Aceton 1 h am Rückfluß gekocht wird. Nach dem Filtrieren und Einengen wird in Ether aufgenommen, dann mit verdünntem wäßrigem Ammoniak und mehrfach mit Wasser gewaschen. Nach Abziehen des Ethers erhält man 11,3 g rohen α-(2-Methylphenyl)-β-methoxyacrylsäuremethylester ($Kp_{0,05}$ 102–108°C).
NMR in $CDCl_3$: 7,53 s 1H
7,16–7,36 bs 4H
3,64 s 3H
3,73 s 3H
2,16 s 3H

Vorschrift B

α-(2-Brommethylphenyl)-β-methoxyacrylsäuremethylester

20,6 g des nach Vorschrift A erhaltenen α-(2-Methylphenyl)-β-methoxyacrylsäuremethylesters, 17,65 g N-Bromsuccinimid, 0,2 g Azodiisobutyronitril und 150 ml $CCl_4$ werden langsam auf 90°C erhitzt. Man hält bei dieser Temperatur, bis alles Succinimid auf dem Lösungsmittel schwimmt. Nach Filtration wird eingeengt, das verbleibende Öl in etwa 5 ml Aceton gelöst und mit n-Hexan zur Kristallisation gebracht. Man erhält 27,5 g farblose Kristalle vom Fp. 86–87°C.
Die folgenden Beispiele erläutern die Herstellung der neuen Wirkstoffe.

Beispiel 2

α-(2-[Phenylthiomethyl]-phenyl)-ß-methoxyacrylsäuremethylester

2 g α-(2-Brommethylphenyl)-ß-methoxyacrylsäuremethylester, 0,65 g Thiophenol und 1,1 g Kaliumcarbonat werden analog Beispiel 1 umgesetzt. Man erhält 1,8 g weiße Kristalle vom Fp. 71°C (Verbindung Nr. 2).

Beispiel 3

α-(2-[Phenylsulfoxymethyl]phenyl)-ß-methoxyacrylsäuremethylester

0,6 g des nach Beispiel 2 erhaltenen α-(2-[Phenylthiomethyl]phenyl)-ß-methoxyacrylsäuremethylesters werden mit 0,39 g 3-Chlorperbenzoesäure in 40 ml Toluol 5 Stunden bei Raumtemperatur gerührt.

Dann fügt man 50 ml Essigester zu und schüttelt 2× mit Natriumhydrogencarbonatlösung und 2× mit Wasser aus. Nach dem Trocknen über $MgSO_4$ und Einengen kristallisiert man aus einem Ether/n-Hexan-Gemisch um. Man erhält 0,56 g vom Fp. 88°C (Verbindung Nr. 31).

Beispiel 4

α-(2-[Phenylsulfonmethyl]phenyl)-ß-methoxyacrylsäuremethylester

1 g des nach Beispiel 2 erhaltenen α-(2-[Phenylthiomethyl]phenyl)-ß-methoxyacrylsäuremethylesters werden analog Beispiel 3 mit 1,4 g 3-Chlorperbenzoesäure in 40 ml Toluol 3 Tage bei Raumtemperatur umgesetzt. Man erhält 1,0 g vom Fp. 135°C (unter Zersetzung) (Verbindung Nr. 4).

In entsprechender Weise lassen sich folgende Verbindungen herstellen:

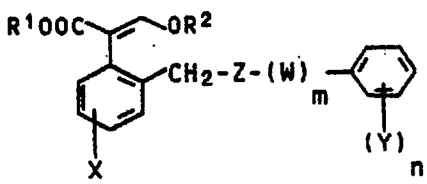

| Nr. | $R^1$ | $R^2$ | X | Z | $W_m$ | $(Y)_n$ | Fp $^0C$/NMR |
|---|---|---|---|---|---|---|---|
| 2 | $CH_3$ | $CH_3$ | H | S | - | H | $71^0$ |
| 3 | $CH_3$ | $CH_3$ | H | SO | - | H | $88^0$ |
| 4 | $CH_3$ | $CH_3$ | H | $SO_2$ | - | H | $135^0$ (Zers.) |
| 5 | $CH_3$ | $CH_3$ | H | O | $-CH_2-$ | H | |
| 6 | $CH_3$ | $CH_3$ | H | O | $-CH_2-CH_2-$ | H | |
| 7 | $CH_3$ | $CH_3$ | H | O | $-CH_2-CH_2-CH_2-$ | H | |
| 8 | $CH_3$ | $CH_3$ | H | O | $-CH_2-CH=CH-$ | H | |
| 9 | $CH_3$ | $CH_3$ | H | O | $-(CH_2)_5-$ | H | |
| 10 | $CH_3$ | $CH_3$ | H | O | $-(CH_2)_7-$ | H | |
| 11 | $CH_3$ | $CH_3$ | H | O | $-(CH_2)_{10}-$ | H | |
| 12 | $CH_3$ | $CH_3$ | H | O | $-CH_2-CH(CH_3)-$ | H | |
| 13 | $CH_3$ | $CH_3$ | H | O | $-CH_2-CH(CH_3)CH_2-$ | H | |
| 14 | $CH_3$ | $CH_3$ | H | O | $-CH(CH_3)CH_2-$ | H | |
| 15 | $CH_3$ | $CH_3$ | H | S | $-CH_2-$ | H | |
| 16 | $CH_3$ | $CH_3$ | H | O | $-CH_2C{\equiv}C-$ | H | |
| 17 | $CH_3$ | $CH_3$ | H | SO | $-CH_2-$ | H | |
| 18 | $CH_3$ | $CH_3$ | H | $SO_2$ | $-CH_2-$ | H | |
| 19 | $CH_3$ | $CH_3$ | H | O | $-CH_2-$ | 4-Cl | |
| 20 | $CH_3$ | $CH_3$ | H | O | $-CH_2-$ | 2,4-$Cl_2$ | |
| 21 | $CH_3$ | $CH_3$ | H | O | $-CH_2-$ | 4-$CH_3$ | |
| 22 | $CH_3$ | $CH_3$ | H | O | $-CH_2-$ | 3-$CH_3$ | |

| Nr. | $R^1$ | $R^2$ | X | Z | $W_m$ | $(Y)_n$ | Fp °C/NMR |
|---|---|---|---|---|---|---|---|
| 23 | $CH_3$ | $CH_3$ | H | O | $-CH_2-CH_2-$ | 4-Cl | |
| 24 | $CH_3$ | $CH_3$ | H | O | $-CH_2-$ | 3-$CF_3$ | |
| 25 | $CH_3$ | $CH_3$ | H | O | $-CH_2-$ | 4-F | |
| 26 | $CH_3$ | $CH_3$ | H | O | - | 2-Cl | |
| 27 | $CH_3$ | $CH_3$ | H | O | - | 2,4-$Cl_2$ | |
| 28 | $CH_3$ | $CH_3$ | H | O | - | 3-Cl | |
| 29 | $CH_3$ | $CH_3$ | H | O | - | 3,4-$Cl_2$ | |
| 30 | $CH_3$ | $CH_3$ | H | O | - | 4-Cl | |
| 31 | $CH_3$ | $CH_3$ | H | O | - | 3,5-$Cl_2$ | |
| 32 | $CH_3$ | $CH_3$ | H | O | - | 2,4,5-$Cl_3$ | |
| 33 | $CH_3$ | $CH_3$ | H | O | - | 4-Br | |
| 34 | $CH_3$ | $CH_3$ | H | O | - | 4-F | |
| 35 | $CH_3$ | $CH_3$ | H | O | - | 4-$OCH_3$ | |
| 36 | $CH_3$ | $CH_3$ | H | O | - | 4-O-n($C_4H_9$) | |
| 37 | $CH_3$ | $CH_3$ | H | O | - | 4-O-t($C_4H_9$) | |
| 38 | $CH_3$ | $CH_3$ | H | O | - | 4-$CH_3$ | |
| 39 | $CH_3$ | $CH_3$ | H | O | - | 2-$CH_3$ | |
| 40 | $CH_3$ | $CH_3$ | H | O | - | 3-$CH_3$ | |
| 41 | $CH_3$ | $CH_3$ | H | O | - | 4-t-$C_4H_9$ | |
| 42 | $CH_3$ | $CH_3$ | H | O | - | 4-cyclo-$C_6H_{11}$ | |
| 43 | $CH_3$ | $CH_3$ | H | O | - | 3-Benzyl | |
| 44 | $CH_3$ | $CH_3$ | H | O | - | 3-Phenoxy | |

| Nr. | $R^1$ | $R^2$ | X | Z | $W_m$ | $(Y)_n$ | Fp °C/NMR |
|---|---|---|---|---|---|---|---|
| 45 | $CH_3$ | $CH_3$ | H | O | - | 4-Phenoxy | |
| 46 | $CH_3$ | $CH_3$ | H | O | - | $3-CH_2OCH_3$ | |
| 47 | $CH_3$ | $CH_3$ | H | O | - | $2-CH_2OCH_3$ | |
| 48 | $CH_3$ | $CH_3$ | H | O | - | $3-CF_3$ | |
| 49 | $CH_3$ | $CH_3$ | H | O | - | $4-CF_3$ | |
| 50 | $CH_3$ | $CH_3$ | H | O | - | $3-C_2H_5$ | |
| 51 | $CH_3$ | $CH_3$ | H | O | - | $2-OCH_3$ | |
| 52 | $CH_3$ | $CH_3$ | H | O | - | $3-OCH_3$ | |
| 53 | $CH_3$ | $CH_3$ | H | O | - | 4-J | |
| 54 | $CH_3$ | $CH_3$ | H | O | - | 3-F | |
| 55 | $CH_3$ | $CH_3$ | H | O | - | 3,4 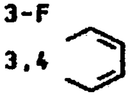 | |
| 56 | $CH_3$ | $CH_3$ | H | O | - | 2,3 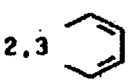 | |
| 57 | $CH_3$ | $CH_3$ | H | O | - | $4-OCHF_2$ | |
| 58 | $CH_3$ | $CH_3$ | H | O | - | $3-OCF_2CHF_2$ | |
| 59 | $CH_3$ | $CH_3$ | H | O | - | $4-SCH_3$ | |
| 60 | $CH_3$ | $CH_3$ | H | O | - | 4-CN | |
| 61 | $CH_3$ | $CH_3$ | H | O | - | 3-CN | |
| 62 | $CH_3$ | $CH_3$ | H | O | - | 4-SCN | |
| 63 | $CH_3$ | $CH_3$ | H | O | - | $4-N(CH_3)_2$ | |
| 64 | $CH_3$ | $CH_3$ | H | O | - | $3-NHCOCH_3$ | |
| 65 | $CH_3$ | $CH_3$ | H | O | - | $3-NHCOOCH_3$ | |

| Nr. | $R^1$ | $R^2$ | X | Z | $W_m$ | $(Y)_n$ | Fp °C/NMR |
|---|---|---|---|---|---|---|---|
| 66 | $CH_3$ | $CH_3$ | H | O | - | $3-NHCON(CH_3)_2$ | |
| 67 | $CH_3$ | $CH_3$ | H | O | - | $4-COOCH_3$ | |
| 68 | $CH_3$ | $CH_3$ | H | O | - | $4-CONHCH_3$ | |
| 69 | $CH_3$ | $CH_3$ | H | O | - | $4-CON(CH_3)_2$ | |
| 70 | $CH_3$ | $CH_3$ | H | O | - | $4-SO_2CH_3$ | |
| 71 | $CH_3$ | $CH_3$ | H | O | - | 4-Phenylsulfonyl | |
| 72 | $CH_3$ | $CH_3$ | H | O | - | $3-COCH_3$ | |
| 73 | $CH_3$ | $CH_3$ | H | O | - | $4-OSO_2CH_3$ | |
| 74 | $CH_3$ | $CH_3$ | H | O | - | $4-SO_2N(CH_3)_2$ | |
| 75 | $CH_3$ | $CH_3$ | H | O | - | 4-CONH- 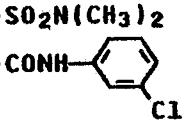 | |
| 76 | $CH_3$ | $CH_3$ | H | O | - | 4-Benzoyl | |
| 77 | $C_2H_5$ | $CH_3$ | H | O | - | H | |
| 78 | $i-C_3H_7$ | $CH_3$ | H | O | - | H | |
| 79 | $n-C_6H_{13}$ | $CH_3$ | H | O | - | H | |
| 80 | $n-C_4H_9$ | $CH_3$ | H | O | - | H | |
| 81 | $n-C_3H_7$ | $CH_3$ | H | O | - | H | |
| 82 | $s-C_4H_9$ | $CH_3$ | H | O | - | H | |
| 83 | $CH_3$ | $C_2H_5$ | H | O | - | H | |
| 84 | $CH_3$ | $i-C_3H_7$ | H | O | - | H | |
| 85 | $CH_3$ | $n-C_6H_{13}$ | H | O | - | H | |
| 86 | $CH_3$ | $n-C_3H_7$ | H | O | - | H | |

| Nr. | $R^1$ | $R^2$ | X | Z | $W_m$ | $(Y)_n$ | Fp °C/NMR |
|---|---|---|---|---|---|---|---|
| 87 | $CH_3$ | s-$C_4H_9$ | H | O | - | H | |
| 88 | $CH_3$ | $CH_3$ | 3-$CH_3$ | O | - | H | |
| 89 | $CH_3$ | $CH_3$ | 4-$CH_3$ | O | - | H | |
| 90 | $CH_3$ | $CH_3$ | 5-Cl | O | - | H | |
| 91 | $CH_3$ | $CH_3$ | 5-Br | O | - | H | |
| 92 | $CH_3$ | $CH_3$ | 6-Cl | O | - | H | |
| 93 | $CH_3$ | $CH_3$ | 6-$CH_3$ | O | - | H | |
| 94 | $CH_3$ | $CH_3$ | 5-F | O | - | H | |
| 95 | $CH_3$ | $CH_3$ | 5-$CF_3$ | O | - | H | |
| 96 | $CH_3$ | $CH_3$ | H | S | - | 4-Cl | |
| 97 | $CH_3$ | $CH_3$ | H | SO | - | 4-Cl | |
| 98 | $CH_3$ | $CH_3$ | H | $SO_2$ | - | 4-Cl | |
| 99 | $CH_3$ | $CH_3$ | H | S | - | 3-$CH_3$ | |
| 100 | $CH_3$ | $CH_3$ | H | S | - | 4-$CH_3$ | |
| 101 | $CH_3$ | $CH_3$ | H | S | - | 2-$CH_3$ | |
| 102 | $CH_3$ | $CH_3$ | H | S | - | 2,4-$Cl_2$ | |
| 103 | $CH_3$ | $CH_3$ | H | O | - | 4$NO_2$ | |
| 104 | $CH_3$ | $CH_3$ | H | O | - | 2Cl,6F | |
| 105 | $CH_3$ | $CH_3$ | H | O | - | 2,3,6$(Cl)_3$ | |
| 106 | $CH_3$ | $CH_3$ | H | O | - | 3,4$(CH_3)_2$ | |
| 107 | $CH_3$ | $CH_3$ | H | O | - | 2-$CH_3$,4t-$C_4H_9$ | |
| 108 | $CH_3$ | $CH_3$ | H | O | - | 2,4$(CH_3)_2$ | |
| 109 | $CH_3$ | $CH_3$ | H | O | - | 2-$NO_2$ | |

| Nr. | $R^1$ | $R^2$ | X | Z | $W_m$ | $(Y)_n$ | Fp °C/NMR |
|---|---|---|---|---|---|---|---|
| 110 | $CH_3$ | $CH_3$ | H | O | - | 4-$C_{12}H_{25}$ | |
| 111 | $CH_3$ | $CH_3$ | H | O | - | 4-$C_2H_5$ | |
| 112 | $CH_3$ | $CH_3$ | H | O | - | 3,4,5$(OCH_3)_3$ | |
| 113 | $CH_3$ | $CH_3$ | H | O | - | 2,5$(CH_3)_2$ | |
| 114 | $CH_3$ | $CH_3$ | H | O | - | 2-F | |
| 115 | $CH_3$ | $CH_3$ | H | O | - | 2CN | |
| 116 | $CH_3$ | $CH_3$ | H | O | - | 2,6-$Cl_2$ | |
| 117 | $CH_3$ | $CH_3$ | H | O | - | 2-Cl,6CN | |
| 118 | $CH_3$ | $CH_3$ | H | O | - | 2-Cl,4$NO_2$ | |
| 119 | $CH_3$ | $CH_3$ | H | O | - | 2,4$(NO_2)_2$ | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten, Phycomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzerkrankungen an verschiedenen Kulturpflanzen oder ihren Samen, insbesondere Weizen, Roggen,

Gerste, Hafer, Reis, Mais, Baumwolle, Soja, Kaffee, Zuckerrohr, Obst und Zierpflanzen im Gartenbau, Weinbau sowie Gemüse – wie Gurken, Bohnen und Kürbisgewächse.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Pyrenophora teres an Gerste,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Alternaria solani an Kartoffeln, Tomaten,
Plasmopara viticola an Reben sowie Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff. Die Aufwandmengen liegen je nach Art des gewünschten Effektes zwischen 0,05 und 3 kg Wirkstoff oder mehr je ha.

Die neuen Verbindungen können auch im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile lösemittelhaltiger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Einige der neuen Verbindungen haben eine sehr gute Wirksamkeit gegen humanpathogene Pilze, wie Trichophyton mentagrophytes und Candida albicans. Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 2 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 3 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 1 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 3 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 2 werden mit 97 Gew.-Teilen feinteiligem Kaolin innig vermischt. Man erhält auf diese Weise ein Stäubemittel, das 3 Gew.-% des Wirkstoffs enthält.

VII. 30 Gew.-Teile der Verbindung Nr. 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 2 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung Nr. 3 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Propylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat,
5-Nitro-isophthalsäure-di-isopropylester,
heterocyclische Strukturen, wie
2-Heptadecyl-2-imidazolin-acetat,
2,4-Dichlor-6-(o-chloranilino)-s-triazin,
O,O-Diethyl-phthalimidophosphonothioat,
5-Amino-1-(bis-(dimethylamino)-phosphinyl)-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarboxylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethylthio-phthalimid
N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon,

Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecy-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
1-(4-Phenylphenoxy)-3,3-dimethyl-1-(1H-1,2,4-triazol-1-yl)-2-butanol
α-(2-Chlorphenyl)-α-(4-chlorphenyl)-5-pyrimidin-methanol
5-Butyl-2-dimethylamino-4-hydroxy-6-methyl-pyrimidin
Bis-(p-Chlorphenyl)-pyridinmethanol,
1,2-Bis-(3-ethoxycarbonyl-2-thioureido)-benzol,
1,2-Bis-(3-methoxycarbonyl)-2-thioureido)-benzol

sowie verschiedene Fungizide, wie

Dodecylguanidinacetat,
3-(3-(3,5-Dimethyl-2-oxycyclohexyl)-2-hydroxyethyl)-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl)-(5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureamid
2-Cyano-N-(ethylaminocarbonyl)-2-methoximino)-acetamid
1-(2-(2,4-Dichlorphenyl)-pentyl)-1H-1,2,4-triazol
2,4'-Difluor-α-(1H-1,2,4-triazolyl-1-methyl)-benzhydrylalkohol
N-(3-Chlor-2,6-dinitro-4-trifluormethylphenyl)-5-trifluormethyl-3-chlor-2-amino-pyridin
1-((bis-(4-Fluorphenyl)-methylsilyl)-methyl)-1H-1,2,4-triazol.

Für die folgenden Versuche wurde als Vergleich die bekannten Wirkstoffe N-Tridecyl-2,6-dimethylmorpholin (A) und sein Acetat (B) verwendet.

<u>Anwendungsbeispiel 1</u>

Wirksamkeit gegen Weizenmehltau

Blätter von in Töpfen gewachsenen Weizenkeimlingen der Sorte "Frühgold" werden mit wäßriger Spritzbrühe, die 80% Wirkstoff und 20% Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80% relativer Luftfeuchtigkeit aufgestellt. Nach 7 Tagen wird das Ausmaß der Mehltauentwicklung ermittelt.

Das Ergebnis des Versuches zeigt, daß der Wirkstoffe 2 bei der Anwendung als 0,025 und 0,0125%ige (Gew.-%) Spritzbrühe eine bessere fungizide Wirkung (90%) zeigt als die bekannten Wirkstoffe A und B (70%).

Anwendungsbeispiel 2

Wirksamkeit gegen Weizenbraunrost

Blätter von in Töpfen gewachsenen Weizensämlingen der Sorte "Frühgold" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95%) gestellt. Während dieser Zeit keimen die Sporen aus und die Keimschläuche dringen in das Blattgewebe ein. Die infizierten Pflanzen werden anschließend mit wäßrigen Spritzbrühen, die 80% Wirkstoff und 20% Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22°C und 65 bis 70% relativer Luftfeuchte aufgestellt. Nach 8 Tagen wird das Ausmaß der Rostpilzentwicklung auf den Blättern ermittelt.

Das Ergebnis des Versuches zeigt, daß bei der Anwendung als 0,025%ige Spritzbrühe beispielsweise die Verbindung 2 eine bessere fungizide Wirkung (93%) hat als der bekannte Wirkstoff A (70%).

Anwendungsbeispiel 3

Wirksamkeit gegen Septoria nodorum

Blätter von in Töpfen gewachsenen Weizenkeimlingen der Sorte "Jubilar" werden mit wäßriger Spritzbrühe, die 80% Wirkstoff und 20% Emulgiermittel in der Trockensubstanz enthält, bis zur Tropfnässe besprüht. Am folgenden Tag werden die angetrockneten Pflanzen mit einer wäßrigen Sporensuspension von Septoria nodorum infiziert und dann für 7 Tage bei 17–19°C und 95%iger relativer Luftfeuchtigkeit weiter kultiviert. Das Ausmaß des Pilzbefalls wird dann visuell ermittelt.

Das Ergebnis des Versuches zeigt, daß bei der Anwendung als 0,05%ige Spritzbrühe beispielsweise die Verbindung 2 eine gute fungizide Wirkung zeigt (97%).

Anwendungsbeispiel 4

Wirksamkeit gegen Plasmopara viticola

Blätter von Topfreben der Sorte "Müller-Thurgau" werden mit wäßriger Spritzbrühe, die 80% Wirkstoff und 20% Emulgiermittel in der Trockensubstanz enthält, besprüht. Um die Wirkungsdauer der Wirkstoffe beurteilen zu können, werden die Pflanzen nach dem Antrocknen des Spritzbelages 8 Tage im Gewächshaus aufgestellt. Erst dann werden die Blätter mit einer Zoosporenaufschwemmung von Plasmopara viticola (Rebenperonospora) infiziert. Danach werden die Reben zunächst für 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit werden die Pflanzen zur Beschleunigung des Sporangienträgerausbruches abermals für 16 Stunden in der feuchten Kammer aufgestellt. Dann erfolgt die Beurteilung des Ausmaßes des Pilzausbruches auf den Blattunterseiten.

Das Ergebnis des Versuches zeigt, daß bei der Anwendung als 0,05 und 0,0125%ige Spritzbrühe beispielsweise die Verbindungen 2 und 3 eine gute fungizide Wirkung zeigen (97%).

**Patentansprüche**

1. Acrylsäureester der allgemeinen Formel

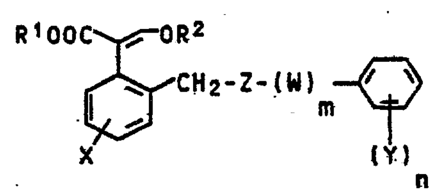

in der $R^1$ und $R^2$ unabhängig voneinander $C_1$–$C_8$-Alkyl bedeuten,
X Wasserstoff, $C_1$–$C_4$-Alkyl, Halogen, $C_1$–$C_4$-Alkoxy, Halogenalkyl, Cyano oder Nitro bedeutet,
W einen gegebenenfalls durch Alkyl substituierten, gegebenenfalls ungesättigten $C_1$–$C_{10}$-Alkylenrest bedeutet,
Y Wasserstoff, Alkyl, Halogenalkyl, Alkoxyalkyl, Cycloalkyl, Aralkyl, Aryl, Aryloxy, Halogen, eine gegebenenfalls substituierte an den Benzolrest annellierte $C_4H_4$-Kette, Alkoxy, Halogenalkoxy, Alkylthio, Thiocyanato, Cyano, $NO_2$,

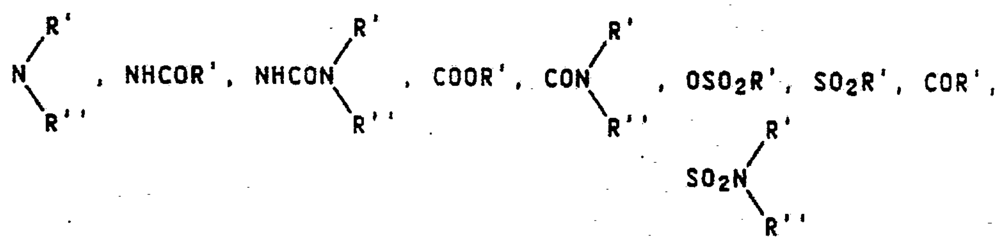

bedeutet, wobei R' und R" jeweils unabhängig voneinander Wasserstoff, Alkyl, Alkoxy, Alkylthio, Cycloalkyl oder gegebenenfalls durch Alkyl, Halogen oder Alkoxy substituiertes Phenyl bedeuten, m die Zahlen 0 oder 1 und n die Zahlen 1 bis 4 sowie Z Sauerstoff, Schwefel, SO oder $SO_2$ bedeutet außer der Verbindung α-(2-Phenoxymethyl-phenyl)-β-methoxy-acrylsäuremethylester.

2. Fungizid, enthaltend einen Trägerstoff und eine Verbindung gemäß Anspruch 1.

3. Verfahren zur Bekämpfung von Pilzen, dadurch gekennzeichnet, daß man die Pilze oder die von Pilzbefall bedrohten Materialien, Pflanzen, Saatgüter oder den Erdboden mit einer fungizid wirksamen Menge einer Verbindung gemäß Anspruch 1 behandelt.

4. α-(2-[Phenylthiomethyl]-phenyl)-β-methoxyacrylsäuremethylester.

5. Fungizid, enthaltend einen Trägerstoff und eine Verbindung gemäß Anspruch 4.

## Claims

1. An acrylate of the formula

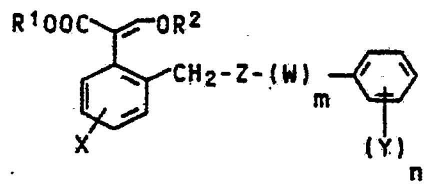

where $R^1$ and $R^2$ independently of one another are each $C_1$–$C_8$-alkyl, X is hydrogen, $C_1$–$C_4$-alkyl, halogen, $C_1$–$C_4$-alkoxy, haloalkyl, cyano or nitro, W is unsubstituted or alkyl-substituted, saturated or unsaturated $C_1$–$C_{10}$-alkylene, Y is hydrogen, alkyl, haloalkyl, alkoxyalkyl, cycloalkyl, aralkyl, aryl, aryloxy, halogen, an unsubstituted or substituted $C_4H_4$ chain which is fused to the benzene radical, alkoxy, haloalkoxy, alkylthio, thiocyanato, cyano, $NO_2$,

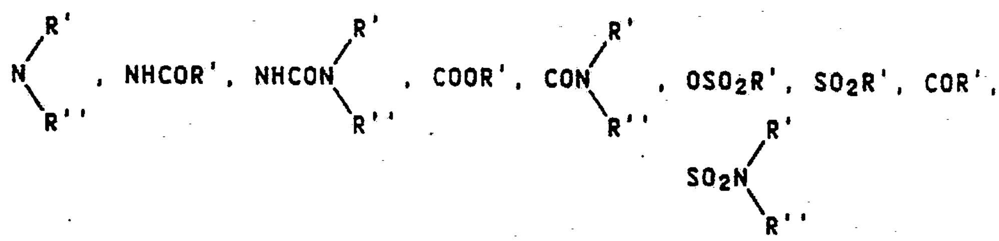

where R' and R" independently of one another are each hydrogen, alkyl, alkoxy, alkylthio or cycloalkyl, or are phenyl which is unsubstituted or substituted by alkyl, halogen or alkoxy, m is 0 or 1, n is from 1 to 4 and Z is oxygen, sulfur, SO or $SO_2$ except methyl α-(2-phenoxymethylphenyl)-β-methoxyacrylate.

2. A fungicide containing a carrier and a compound as claimed in claim 1.

3. A process for combating fungi, wherein the fungi or the materials, plants, seeds or soil threatened by fungus attack are treated with a fungicidally effective amount of a compound as claimed in claim 1.

4. Methyl α-(2-phenylthiomethylphenyl)-β-methoxy-acrylate.

5. A fungicide containing a carrier and the compound claimed in claim 4.

## Revendications

1. Ester d'acide acrylique de la formule générale

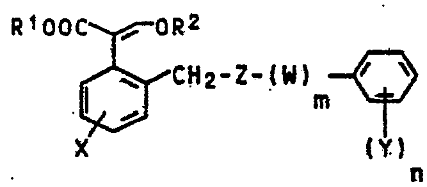

dans laquelle $R^1$ et $R^2$ représentent, indépendamment l'un de l'autre, alkyle en $C_1$–$C_8$,
X représente hydrogène, alkyle en $C_1$–$C_4$ halogène, alcoxy en $C_1$–$C_4$, halogénoalkyle, cyano ou nitro,
W représente un reste alkylène en $C_1$–$C_{10}$, éventuellement insaturé, éventuellement substitué par alkyle,
Y représente hydrogène, alkyle, halogénoalkyle, alcoxyalkyle, cycloalkyle, aralkyle, aryle, aryloxy, halogène, une chaîne $C_4H_4$ condensée, éventuellement substituée sur le reste benzène, alcoxy, halogénoalcoxy, alkylthio, thiocyanato, cyano, $NO_2$

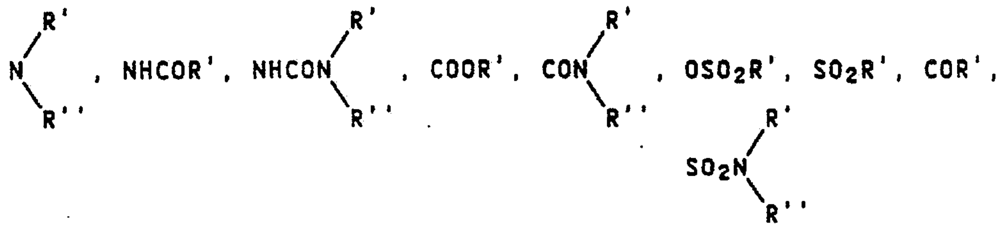

R' et R" représentent chacun, indépendamment l'un de l'autre, hydrogène, alkyle, alcoxy, alkylthio, cycloalkyle ou phényle éventuellement substitué par alkyle, halogène ou alcoxy,
m représente le nombre 0 ou 1 et n les nombres 1 à 4 et Z représente oxygène soufre, SO ou $SO_2$.

2. Fongicide contenant un support et un composé selon la revendication 1.

3. Procédé pour lutter contre les champignons, caractérisé par le fait que l'on traite les champignons ou les matériaux, plantes, semences ou le sol, menacés par les champignons, avec une quantité efficage du point de vue fongicide d'un composé selon la revendication 1.

4. Ester méthylique de l'acide α-(2-phénylthiométhylphényl)-β-méthoxyacrylique.

5. Fongicide contenant un support et un composé selon la revendication 4 à l'exception du composé ester méthylique de l'acide α-(2-phénoxyméthyl-phényl)-β-méthoxyacrylique.